# EUROPEAN PATENT APPLICATION

(11) **EP 3 327 147 A1**
(43) Date of publication of application: **30.05.2018**
(21) Application number: 17204174.1
(22) Date of filing: 28.11.2017
(51) Int. Cl.: C12Q 1/6825

(54) **METHOD FOR QUANTITATIVELY PROFILING NUCLEIC ACIDS**

(30) Priority: 29.11.2016 US 201662427581 P
(71) Applicant: Helios Bioelectronics Inc., Zhubei City, 30261 (TW)
(72) Inventor: CHAN, Hardy Wai-Hong, Redwood City, CA 94065 (US); YANG, Yuh-Shyong, 300 Hsinchu (TW); CHEN, Wen-Yih, 320 Taoyuan (TW); LIN, Ming-Yu, 310 Zhudong Township (TW)
(74) Representative: Fuchs Patentanwälte Partnerschaft mbB

(57) **Abstract**

The present invention relates to a method for establishing a quantitative landscape of a group of target nucleic acid molecules. The method comprises conducting a plurality of hybridization reactions for quantifying each target nucleic acid molecule of the group of target nucleic acid molecules to generate a plurality of quantitative signals; generating ratios between two quantitative signals; and consolidating the ratios for constructing the quantitative landscape of the group of target nucleic acid molecules. The method according to the invention is able to profile numerous target nucleic acid molecules to provide a big data standardization means for a variety of applications with high sensitivity and wide dynamic range.

## Description

### FIELD OF THE INVENTION

This invention relates to a molecular detection technique. More specifically, the invention relates to a method for quantitatively profiling nucleic acid molecules and thereby for biological and clinical applications.

### BACKGROUND OF THE INVENTION

Molecular detection plays an important role in clinical diagnosis and molecular biology research. Several systems have been developed to perform molecular detection for detecting and/or identifying a target molecule in a sample. Generally, molecular events in an organism are instigated by a numbers of molecules instead of a single one, and thus, profiling a group of molecules should be far more important than detecting and/or identifying a single molecule within a biological sample.

Several detection procedures for profiling a group of molecules have been developed and most often based on methods generally referred to as microarrays. Conventional microarray methods require labeled molecules. However, drawbacks regarding the need for labeling have rendered microarrays not the most versatile and convenient quantitative method for profiling a group of molecules. Moreover, due to such complexities, false positive and negative results have been a common problem in many applications.

Moreover, due to the scarcity of a subset of molecules, such as in the case of microRNAs (miRNAs), it is challenging to quantify all molecules present in a particular sample. A case-in point was shown in "Direct Quantification of Circulating miRNAs in Different Stages of Nasopharyngeal Cancerous Serum Samples in Single Molecule Level with Total Internal Reflection Fluorescence Microscopy, See-Lok Ho, Ho-Man Chan, Amber Wai-Yan Ha, Ricky Ngok-Shun Wong, and Hung-Wing Li, Anal. Chem., 2014, 86 (19), pp 9880-9886)". Even though there are methods developed for the quantification of these rare molecules, they require enzymatic/chemical labeling or enzyme amplification (Direct detection and quantification of microRNAs, Eric A. Hunt, Ann M. Goulding, and Sapna K. Deo; Anal Biochem., 2009 Apr 1; 387(1): 1-12; Absolute quantification of microRNAs by using a universal reference, Ute Bissels, Stefan Wild, Stefan Tomiuk, Angela Holste, Markus Hafner, Thomas Tuschl, and Andreas Bosio, RNA, 2009 Dec; 15(12): 2375-2384; Direct quantification of microRNA at low picomolar level in sera of glioma patients using a competitive hybridization followed by amplified voltammetric detection, Jianxiu Wang, Xinyao Yi, Hailin Tang, Hongxing Han, Minghua Wu, and Feimeng Zhou, Anal. Chem., 2012, 84 (15), pp 6400-6406; Direct quantification of circulating miRNAs in different stages of nasopharyngeal cancerous serum samples in single molecule level with total internal reflection fluorescence microscopy, See-Lok Ho, Ho-Man Chan, Amber Wai-Yan Ha, Ricky Ngok-Shun Wong, and Hung-Wing Li, Anal. Chem., 2014, 86 (19), pp 9880-9886; Quantitative and stoichiometric analysis of the microRNA content of exosomes, John R. Chevillet, Qing Kang, Ingrid K. Ruf, Hilary A. Briggs, Lucia N. Vojtech, Sean M. Hughes, Heather H. Cheng, Jason D. Arroyo, Emily K. Meredith, Emily N. Gallichotte, Era L. Pogosova-Agadjanyan, Colm Morrissey, Derek L. Stirewalt, Florian Hladik, Evan Y. Yu, Celestia S. Higano, and Muneesh Tewari, PNAS, 2014, 111(4), pp 14888-14893). It has been well appreciated that the amplification step is a source of artifacts. All primers are not equally utilized and the bias is increasingly exaggerated as the number of amplification cycle goes up. The steps of chemical labeling or enzyme amplifying are laborious, as well as time and cost-consuming. Furthermore, the signal transformation of chemical labeling or enzyme amplifying causes errors in the detection. The disadvantages may be acceptable in quantifying a single or just a few miRNA molecule; however, it is impossible to apply the conventional processes for profiling a larger group of miRNA molecules or, in general, nucleic acid molecules.

### SUMMARY OF THE INVENTION

The invention is to provide a method for establishing a quantitative landscape of a group of target nucleic acid molecules comprising:
conducting a plurality of hybridization reactions for quantifying each target nucleic acid molecule of the group of target nucleic acid molecules to generate a plurality of quantitative signals; wherein the plurality of hybridization reactions have quantitation limits lower than about 1 fM;
generating plural of ratios between any two quantitative signals; and
consolidating the ratios for constructing the quantitative landscape of the group of target nucleic acid molecules.

The method according to the invention is able to dynamically monitor a quantitative landscape the numerous target nucleic acid molecules to provide a big data standardization means for a variety of applications. The method is able to provide an internal standardization approach by self-reference, and determine the ratio of nucleic acid molecules, such as miRNA, from at least two different samples.

The invention is to provide a quantitative landscape of a group of target nucleic acid molecules, which is established with the method as mentioned above.

The present invention is described in detail in the following sections. Other characteristics, purposes and advantages of the present invention can be found in the detailed description and claims.

### BRIEF DESCRIPTION OF THE DRAWINGS

FIG. 1 shows one preferred embodiment of an electrically neutral nucleotide in a partially neutral single-stranded oligonucleotide according to the invention.
FIG. 2 shows Id-Vg curve of miR-885-5p with (A) probe-885-5p; (B) probe-579-3p; and (C) probe-107.
FIG. 3 shows Id-Vg curve of miR-579-3p with (A) probe-885-5p; (B) probe-579-3p; and (C) probe-107.
FIG. 4 shows Id-Vg curve of miR-107 with (A) probe-885-5p; (B) probe-579-3p; and (C) probe-107.
FIG. 5 shows the threshold voltage shift of NWFET induced by different targets and probes.
FIG. 6 shows the standard curves of using NWFET as an electronic biosensor to quantify the absolute amount of varies miRNA within PC3 cells.
FIG. 7 shows the standard curves of using NWFET as an electronic biosensor to quantify the absolute amount of varies miRNA within CWR cells.
FIG. 8 shows the dynamic range of using NWFET as an electronic biosensor spanned for seven orders from 0.0179 fM to 179000 fM of miR-301a.
FIG. 9 shows the Ct value of 0.0179 fM to 179000 fM of miR-301a by q-PCR.
FIG. 10 shows the ratio of miR-21 concentration versus another 6 miRNA in PC3 cells.
FIG. 11 shows the ratio of miR-33 concentration versus another 6 miRNA in PC3 cells.
FIG. 12 shows the ratio of miR-301a concentration versus another 6 miRNA in PC3 cells.
FIG. 13 shows the ratio of miR-21 concentration versus another 6 miRNA in CWR cells.
FIG. 14 shows the ratio of miR-33 concentration versus another 6 miRNA in CWR cells.
FIG. 15 shows the ratio of miR-301a concentration versus another 6 miRNA in CWR cells.
FIG. 16 shows 3D plot of the ratio for miR-21, 301a, 33, 34a, 107, 375, 141 with randomly selecting two miRNA within these 7 miRNA in PC3 cells.
FIG. 17 shows 3D plot of the ratio for miR-21, 301a, 33, 34a, 107, 375, 141 with randomly selecting two miRNA within these 7 miRNA in CWR cells.

### DETAILED DESCRIPTION OF THE INVENTION

The invention is to provide a method for establishing a quantitative landscape of a group of target nucleic acid molecules comprising:
conducting a plurality of hybridization reactions for quantifying each target nucleic acid molecule of the group of target nucleic acid molecules to generate a plurality of quantitative signals; wherein the plurality of hybridization reactions have quantitation limits lower than about 1 fM;
generating plural of ratios between any two quantitative signals; and
consolidating the ratios for constructing the quantitative landscape of the group of target nucleic acid molecules.

In one embodiment of the invention, the quantitative landscape indicates directly or indirectly the relationship between members in the group of target nucleic acid molecules or between members in the group of target nucleic acid molecules and other related biological molecules. The quantitative landscape can be further processed or analyzed as needed. The quantitiative landscape according to the invention can be taken as a marker for representing a specific condition of a subject such as a disease. Preferably, several quantitative landscapes of several specific conditions are established, respectively, and each quantitative landscape is stored as an index of the corresponding specific condition for diagnosis.

Preferably, the method according to the invention is absent from labeling in hybridization reaction; that is, the method according to the invention does not employ labeling. The labeling according to the invention refers to a process for adding an element for identifying or quantifying the presence of the target nucleic acid molecule. In one embodiment of the invention, the element is attached to the target nucleic acid molecule, and in another embodiment of the invention, the element is attached to a probe for hybridizing the target nucleic acid molecule. The element for labeling is able to produce a signal for identifying the presence of the target nucleic acid molecule. Examples of the signal include but are not limited to a fluorescence signal, a luminescence signal, a visible light signal, and an isotope signal. Examples of the element include but are not limited to a chemical agent, a fluorescence dye, a luminescence dye, a biomolecule, and an isotope dye.

Preferably, the method according to the invention is absent from enzyme amplifying in hybridization reaction; that is, the method according to the invention does not employ amplifying. The enzyme amplifying according to the invention refers to a process for amplifying the abundance of the target nucleic acid molecules by an enzyme. In one embodiment of the invention, the enzyme is attached to the target nucleic acid molecules, and the enzyme is able to amplify the target nucleic acid molecules, such in a polymerase chain reaction.

As used herein, the term "an oligonucleotide" or "a nucleic acid" refers to an oligomer or polymer of nucleotides. The term "nucleotide" refers to an organic molecule composed of a nitrogenous base, a sugar, and one or more phosphate groups; preferably one phosphate group. The nitrogenous base includes a derivative of purine or pyrimidine. The purine includes substituted or unsubstituted adenine and substituted or unsubstituted guanine; the pyrimidine includes substituted or unsubstituted thymine, substituted or unsubstituted cytosine and substituted or unsubstituted uracil. The sugar is preferably a five-carbon sugar, more preferably substituted or unsubstituted ribose or substituted or unsubstituted deoxyribose. The phosphate groups form bonds with the 2, 3, or 5-carbon of the sugar; preferably, with the 5-carbon site. For forming the oligonucleotide, the sugar of one nucleotide is joined to the adjacent sugar by a phosphodiester bridge. Preferably, the nucleic acid is DNA, cfDNA, methylated DNA, mRNA, miRNA, LncRNA, and ribosomal RNA; more preferably, miRNA.

As used herein, the term "a target nucleic acid molecule" refers to a naturally occurring or artificial molecule. In another aspect, the target nucleic acid molecule is purified or mixed with other contents.

In a preferred embodiment of the invention, the target nucleic acid molecule may include DNA, cfDNA, methylated DNA, mRNA, miRNA, LncRNA, and ribosomal RNA; more preferably, miRNA.

In one embodiment of the invention, the target nucleic acid molecule is linked to a biomolecule. As used herein, the term "a biomolecule" refers to a specified small molecule or a macromolecule that links to the target nucleic acid molecule. Preferably, the biomolecule is a macromolecule such as a protein, peptide, or polysaccharide; more preferably, a protein. The biomolecule is naturally occurring or artificial. In one preferred embodiment of the invention, the expression pattern of the biomolecule is different in a normal condition and in an abnormal condition, such as a disease. In another preferred embodiment of the invention, the expression pattern of the biomolecule is different in different cell types. In yet another preferred embodiment of the invention, the biomolecule is an antibody, an antigen, an enzyme, a substrate, a ligand, a receptor, a cell membrane-associated protein, or a cell surface marker.

The target nucleic acid molecule can be a single-stranded molecule or a double-stranded molecule. The manner of obtaining the single strand of the double-stranded target nucleic acid molecule can be, for example, heating or changing ion strength of the environment of the double-stranded target nucleic acid molecule.

Preferably, the group of target nucleic acid molecules contains at least three target nucleic acid molecules; preferably, at least 4, 5, 6, 7, 8, 9, 10, 11, 12, 13, 14, 15, 20, 25, 30, 35, 40, 45, 50, 60, 70, 80, 90, or 100 target nucleic acid molecules. In one preferred embodiment of the invention, a quantitative landscape of the group of target nucleic acid molecules is different in a normal condition and in an abnormal condition, such as a disease. In another preferred embodiment of the invention, a quantitative landscape of the group of target nucleic acid molecules is different in different cell types.

As used herein, the term "a quantitative landscape of a group of target nucleic acid molecules" refers to a quantitative profile or pattern of the group of target nucleic acid molecules, including but not limited to a combination of a content/concentration of each target nucleic acid molecule in the group, or a combination of ratios of a content/concentration of each target nucleic acid molecule in the group. Preferably, the quantitative landscape focuses on a whole picture of the amounts of the group of target nucleic acid molecules rather than individual amount thereof.

The method according to the invention comprises conducting a plurality of hybridization reactions for quantifying each target nucleic acid molecule of the group of target nucleic acid molecule; and preferably, the plurality of the hybridization reactions are conducted simultaneously. In one preferred embodiment of the invention, the target nucleic acid molecules are integrated in one chip or microarray.

The hybridization reaction according to the invention is ultrasensitive. Preferably, the plurality of hybridization reactions have quantitation limits lower than about 1 fM. The hybridization reaction is able to quantify a target nucleic acid molecule at the concentration lower than about 1 fM in a sample; preferably lower than about 0.01 fM; more preferably lower than about 0.001 fM. Depending upon such ultrasensitive hybridization reactions, the target nucleic acid molecules with ultralow concentrations such as miRNA are successfully quantified.

In one embodiment of the invention, the difference between the smallest and largest quantitative signals (also known as dynamic range) is at least two orders of magnitude; more preferably, at least four orders of magnitude; still more preferably, at least seven orders of magnitude.

The method according to the invention comprises obtaining a signal change occurring due to the hybridization reaction for identifying each target nucleic acid molecule in the group. According to the invention, if a target nucleic acid molecule is present, the hybridization reaction occurs. The hybridization reaction forms a duplex of an oligonucleotide molecule probe with the target nucleic acid molecule according to base complementarity.

Several types of quantitative signal can be generated in the hybridization reaction according to the invention. Preferably, the plurality of quantitative signals are selected from the group consisting of an electrical change, a weight change, absorbance wavelength change, absorbance intensity change, fluorescence and fluorescence intensity change, and a reflective index change, more preferably, an electrical change. Because the oligonucleotide molecule carries electrical charges, an electrical change occurs due to the hybridization reaction by introducing a single-stranded nucleic acid molecule. By monitoring of the electrical changes, the presence and content of the target nucleic acid molecule is detected.

The electrical change according to the invention includes but is not limited to increase of electrical charges. The electrical change can be detected as an electrical signal. The electrical signal includes but is not limited to an electric charge change, an electric current change, an electric resistance change, a threshold voltage shift change, an electric conductivity change, an electric field change, an electric capacitance change, an electric current change, an electron change, and an electron hole change. In one preferred embodiment of the invention, the electrical change is a threshold voltage shift change.

In one embodiment of the invention, the quantitative signal according to the invention is detected with a detector. According to different types of quantitative signal, different types of detector are provided. For example, the detector is means for detecting an electrical change, a weight change, absorbance wavelength change, absorbance intensity change, fluorescence and fluorescence intensity change, or a reflective index change. Preferably, the detector according to the invention is not only able to detect the presence of the change, but also convert the change to values presenting the magnitude of the change. Examples of the detector include but are not limited to a transistor, resonance instrument, or spectrometer.

Preferably, a single strand of the target nucleic acid molecule is hybridized by a recognizing single-stranded oligonucleotide molecule to form a duplex according to base complementarity.

As used herein, the term "a recognizing single-stranded oligonucleotide molecule" refers to a single-stranded oligonucleotide molecule able to form a duplex with the target nucleic acid molecule according to base complementarity. In other words, the recognizing single-stranded oligonucleotide molecule acts as a probe to hybridize the target nucleic acid molecule. The duplex preferably refers to a double-stranded structure, and in which strand is the single strand of the target nucleic acid molecule and the other strand is the recognizing single-stranded oligonucleotide molecule as a probe. Preferably, the recognizing single-stranded oligonucleotide molecule has a sequence matched to that of the target nucleic acid molecule; more preferably, has a sequence perfectly matched to that of the target nucleic acid molecule. By forming the duplex, the target nucleic acid molecule can be captured from a mixture in a sample. The capturing step also refers to a purification step of specifically selecting the target nucleic acid molecule and presenting the target nucleic acid molecule in the duplex.

According to the invention, the method is for establishing a quantitative landscape of a group of target nucleic acid molecules in a sample. The sample according to the invention is derived from a naturally occurring origin or derived from artificial manipulation. Preferably, the sample is derived from a naturally occurring origin such as an extract, body fluid, tissue biopsy, liquid biopsy, or cell culture. In another aspect, the sample is processed according to the reaction of detection. For example, the pH value or ion strength of the sample may be adjusted.

The recognizing single-stranded oligonucleotide molecule according to the invention may be presented in a solution or attached to a solid surface. Preferably, the recognizing single-stranded oligonucleotide molecule is attached to a solid surface or the recognizing single-stranded oligonucleotide molecule is spaced apart from the solid surface by a distance.

As used herein, the term "solid surface" refers to a solid support including but not limited to a silicon, silicon oxide, polymer, paper, fabric, or glass. Preferably, the solid surface to be employed varies depending on an electrical change detecting element as mentioned below. For example, when the method adopts a field-effect transistor to detect the electrical change, the solid surface is a transistor surface of the field-effect transistor; when the method adopts a surface plasmon resonance, the solid surface is a metal surface of a surface plasmon resonance.

Preferably, the solid surface is coupled with an electrical change detecting element for detecting the electrical change. The electrical change detecting element is applied for detecting whether the electrical change occurs. Preferably, the electrical change detecting element is a field-effect transistor or a surface plasmon resonance.

In a preferred embodiment of the invention, the material of the solid surface is silicon; preferably polycrystalline silicon or single crystalline silicon; more preferably polycrystalline silicon. Polycrystalline silicon is cheaper than single crystalline silicon, but because the polycrystalline has more grain boundary, a defect usually occurs in the grain boundary that hinders electron transduction. Such phenomenon makes the solid surface uneven and quantification difficult. Furthermore, ions may penetrate into the grain boundary of the polycrystalline and cause detection failure in solution. In addition, polycrystalline silicon is not stable in air. The abovementioned drawbacks, however, would not interfere with the function of the method according to the invention.

The manner of attaching the recognizing single-stranded oligonucleotide molecule and the solid surface depends on the material of the solid surface and the type of recognizing single-stranded oligonucleotide molecule. In one embodiment of the invention, the recognizing single-stranded oligonucleotide molecule links to the solid surface through a covalent bond. Examples of the covalent bond include but are not limited to the following methods, depending on the solid surface chemistry and the modification of the oligonucleotide. In one embodiment of the invention, when silicon oxide is used as the solid surface, the solid surface is modified by using (3-Aminopropyl)triethoxysilane (APTES). The silicon atom in the molecule of APTES performs a covalent bond with the oxygen atom of the hydroxyl group and it converts the surface's silanol groups (SiOH) to amines; then the 5'-amino group of recognizing single-stranded oligonucleotide molecule is covalently bonded with the solid surface amines group by glutaraldehyde (Roey Elnathan, Moria Kwiat, Alexander Pevzner, Yoni Engel, Larisa Burstein Artium Khatchtourints, Amir Lichtenstein, Raisa Kantaev, and Fernando Patolsky, Biorecognition Layer Engineering: Overcoming Screening Limitations of Nanowire-Based FET Devices, Nano letters, 2012, 12, 5245 - 5254). In another embodiment of the invention, the solid surface is modified into self-assembling monolayer molecules attaching physically, and chemically to the surface, but not limit to with different functional groups for covalently linking to different functional groups of the recognizing single-stranded oligonucleotide molecule by various chemical reactions (Srivatsa Venkatasubbarao, Microarrays - status and prospects, TRENDS in Biotechnology Vol. 22 No. 12 December 2004; Ki Su Kim, Hyun-Seung Lee, Jeong-A Yang, Moon-Ho Jo and Sei Kwang Hahn, The fabrication, characterization and application of aptamer-functionalized Si-nanowire FET biosensors, Nanotechnology 20 (2009)).

In another preferred embodiment of the invention, the recognizing single-stranded oligonucleotide molecule is spaced apart from the solid surface by a distance. Since the electrical change detecting element is applied for detecting the electrical change, the recognizing single-stranded oligonucleotide molecule does not necessarily need to directly bind to the solid surface, provided that the distance between the recognizing single-stranded oligonucleotide molecule and the solid surface is small enough to allow the electrical change detecting element to detect the electrical change. Preferably, the distance between the solid surface and the recognizing single-stranded oligonucleotide molecule is about 0 to about 10 nm; more preferably about 0 to about 5 nm, when the hybridization efficiency is not interfered.

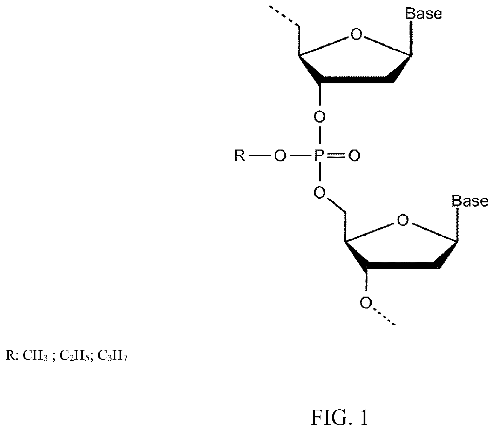

In one preferred embodiment of the invention, the recognizing single-stranded oligonucleotide molecule is a partially neutral single-stranded oligonucleotide comprising at least one electrically neutral nucleotide and at least one negatively charged nucleotide. The manner of rendering a nucleotide electrically neutral is not limited. In one embodiment of the invention, the electrically neutral nucleotide comprises a phosphate group substituted by an alkyl group. Preferably, the alkyl group is a C₁-C₆ alkyl group; more preferably, the alkyl group is a C₁-C₃ alkyl group. Examples of the C₁-C₃ alkyl group include but are not limited to methyl, ethyl and propyl. FIG. 1 shows one preferred embodiment of the electrically neutral nucleotide of the partially neutral single-stranded oligonucleotide according to the invention. The negatively-charged oxygen atom in the phosphate group is changed to a neutral atom without charge. The way to substitute the phosphate group with the alkyl group can be selected from common chemical reactions.

The negatively charged nucleotide according to the invention comprises a phosphate group with at least one negative charge. The unmodified nucleotide is preferably a naturally occurring nucleotide without modification or substitution. In one preferred embodiment of the invention, the negatively charged nucleotide comprises an unsubstituted phosphate group.

The partially neutral single-stranded oligonucleotide according to the invention is partially rendered electrically neutral. The sequence or length is not limited, and can be designed according to a target nucleic acid molecule based on the disclosure of the invention.

The number of electrically neutral nucleotides and negatively charged nucleotides depend on the sequence of the partially neutral single-stranded oligonucleotide and the condition under the duplex formation. The positions of the electrically neutral nucleotides and negatively charged nucleotides also depend on the sequence of the partially neutral single-stranded oligonucleotides and the condition under the duplex formation. The number and positions of the electrically neutral nucleotides and negatively charged nucleotides can be designed according to available information based on the disclosure of the invention. For example, the number and positions of the electrically neutral nucleotides can be designed by molecular modeling calculation based on double stranded (ds) structural energy, and the melting temperature (Tm) of dsDNA/DNA or dsDNA/RNA can then be determined by reference to the structural energy.

In one preferred embodiment of the invention, the partially neutral single-stranded oligonucleotide comprises a plurality of the electrically neutral nucleotides, and at least one negatively charged nucleotide is positioned between two of the electrically neutral nucleotides; more preferably, at least two negatively charged nucleotides are positioned between two of the electrically neutral nucleotides.

By introducing the electrically neutral nucleotide, the melting temperature difference between perfect match double-stranded oligonucleotides and mismatched double-stranded oligonucleotides of the partially neutral single-stranded oligonucleotide according to the invention is higher compared with that of a conventional DNA probe. Without being restricted by theory, it is surmised that the electrostatic repulsion force between two strands is lowered by introducing the neutral oligonucleotide, and the melting temperature is raised thereby. By controlling the number and positions of electrically neutral nucleotides, the melting temperature difference is adjusted to a desired point, providing a better working temperature or temperature range to differentiate the perfect and mismatched oligonucleotides, thereby improving capture specificity. Such design benefits consistency of the melting temperature of different partially neutral single-stranded oligonucleotides integrated in one chip or array. The number of reactions to be detected can be raised dramatically with high specificity and more detection units can be incorporated into a single detection system. The design provides better microarray operation conditions.

In one preferred embodiment of the invention, the partially neutral single-stranded oligonucleotide comprises a first portion attached to the solid surface; the length of the first portion is about 50% of the total length of the partially neutral single-stranded oligonucleotide; and the first portion comprises at least one electrically neutral nucleotide and at least one negatively charged nucleotide; more preferably, the length of the first portion is about 40% of the total length of the partially neutral single-stranded oligonucleotide; still more preferably, the length of the first portion is about 30% of the total length of the partially neutral single-stranded oligonucleotide.

In one preferred embodiment of the invention, the partially single-stranded nucleotide further comprises a second portion adjacent to the first portion. The second portion is located in the distal end to the solid surface. The second portion comprises at least one electrically neutral nucleotide and at least one negatively charged nucleotide. The description of the electrically neutral nucleotide and the negatively charged nucleotide is the same as that of the first portion and is not repeated herein.

In one preferred embodiment of the invention, the method is performed in a buffer lower than about 100 mM; more preferably, lower than about 80 mM, 50 mM, 40 mM, 30 mM, 20 mM or 10 mM. Without being restricted by theory, it is surmised that by applying the partially neutral single-stranded oligonucleotide, the duplex formed between the partially neutral single-stranded oligonucleotide with the target nucleic acid molecule can happen without the need to suppress the electrostatic repulsive forces between the partially charged semi-neutral single-stranded oligonucleotide and its target. The hybridization is then driven by the base pairing and the stacking force of each strand. Consequently, the duplex can be formed at a lower salt condition. With FET, the lower ion strength increases the detection length (the debye length) and, in turn, enhances the detection sensitivity.

In one embodiment of the invention, the improved hybridization specificity for forming the duplex can be seen mainly in two aspects of FET detection compared to a conventional detection. First, the melting temperature difference is higher. Second, the buffer has a lower salt condition, and the FET detection length (the debye length) is greater. Both of these differences result in improvement of detection sensitivity.

In a preferred embodiment of the invention, several recognizing single-stranded oligonucleotide molecules are contained in one system to carry out several detections in one manipulation. For example, a plurality of recognizing single-stranded oligonucleotide molecules may be incorporated in a detection system. Preferably, the detection system is a microarray or a chip.

The method according to the invention comprises generating ratios between any two quantitative signals. In one embodiment according to the invention, the number of the target nucleic acid molecules in the group of target nucleic acid molecules is *n,* and the number of ratios to be correlated is [*n* x (*n*-1) / 2]; wherein n is an integer. Without intending to be limited by theory, the inventors of the present disclosure believe that if *n* is larger, the quantitative landscape can provide more precise information for an interested condition.

The manner of generating the ratios between two quantitative signals includes but is not limited to transmitting the quantitative signals and calculating the ratios. In one embodiment of the invention, the quantitative signals are transmitted from the detector to a computer for storing the quantitative signals and performing the calculations. Preferably, the quantitative signals have been converted into values by the detector as mentioned above. In another aspect, the ratios of quantitative signals are determined by dividing one quantitative signal with one another quantitative signal. Preferably, one quantitative signal is divided with all other quantitative signals, and all quantitative signals are processed through such division to obtain a ratio group.

According to the invention, the method comprises consolidating the ratios for constructing the quantitative landscape of the group of target nucleic acid molecules. The manner of consolidating and constructing includes but is not limited to plotting the ratio group into one diagram representing every ratio in the ratio group. Such consolidating and constructing can be performed by a computer with commercialized software. For example, the ratios are consolidated as a 3-D plot.

Combinations of the ratios are provided as a quantitative landscape of the target nucleic acid molecules in the group to provide a big data standardization means for a variety of applications.

The invention is to provide a quantitative landscape of a group of target nucleic acid molecules, which is established with the method as mentioned above.

Preferably, the quantitative landscapes according to the invention are established under different conditions for comparing the different quantitative landscapes between different conditions.

The following examples are provided to aid those skilled in the art in practicing the present invention.

### EXAMPLES

### Synthesis of partially neutral single-stranded oligonucleotide as recognizing single-stranded oligonucleotide molecule:

Deoxy cytidine (n-ac) p-methoxy phosphoramidite, thymidine p-methoxy phosphoramidite, deoxy guanosine (n-ibu) p-methoxy phosphoramidite, and deoxy adenosine (n-bz) p-methoxy phosphoramidite (all purchased from ChemGenes Corporation, USA) were used to synthesize an oligonucleotide according to a given sequence based on solid-phase phosphotriester synthesis or by Applied Biosystems 3900 High Throughput DNA Synthesizer (provided by Genomics® Biosci & Tech or Mission Biotech).

The synthesized oligonucleotide was reacted with weak alkaline in toluene at room temperature for 24 hours, and the sample was subjected to ion-exchange chromatography to adjust the pH value to 7. After the sample was concentrated and dried, the partially neutral single-stranded oligonucleotide was obtained.

### Recognizing single-stranded oligonucleotide molecule attachment:

Recognizing single-stranded oligonucleotide molecule attachment was performed by functionalization of the SiNW surface layer (SiO₂). First, (3-Aminopropyl)triethoxysilane (APTES) was used to modify the surface. The silicon atom in the molecule of APTES performed a covalent bond with the oxygen of the hydroxyl group and converted the surface's silanol groups (SiOH) to amines. Samples were immersed in 2% APTES (99% EtOH) for 30 minutes and then heated to 120°C for 10 min. After this step, amino groups (NH₂) were the terminal units from the surface.

Next, glutaraldehyde was used as a grafting agent for DNA immobilization. Glutaraldehyde binding was achieved through its aldehyde group (COH) to ensure a covalent bond with the amino group of APTES. For this step, samples were immersed in 2.5 % glutaraldehyde (10mM sodium phosphate buffer) in liquid for 1 hour at room temperature. For probe immobilization, 5'-amino group of DNA strands were linked to the aldehyde groups of the linker. A 500 µL drop solution of 1 µmol DNA probes was deposited onto the NWs for 18 hours.

### Generating a plurality of quantitative signals

The sequences of the recognizing single-stranded oligonucleotide molecule (probe) and the target oligonucleotide molecule (target) are listed in Table 1.

**Table 1:**

| Sequence name | DNA sequence (5'→3') | SEQ ID NO.: |
|---|---|---|
| Probe-885-5p(nDNA-p4) |   n      n      n      n A GAG GCA GGG TAGTGTAATGGA | 1 |
| Probe-579-3p(nDNA-p4) |   n      n      n      n A ATC GCG GTT TATACCAAATGA | 2 |
| Probe-107(nDNA-p4) | n n n n T GAT AGC CCT GTACAATGCTGCT | 3 |
| hsa-miR-21-5p |  n   n   n   n T CAA CAT CAG TCTGATAAGCTA | 4 |
| hsa-miR-301a-3p | n n n n G CTT TGA CAA TACTATTGCACTG | 5 |
| hsa-miR-34a-5p | n n n A CAA CCA GCTAAGACACTGCCA | 6 |
| hsa-miR-375 | n n n n T CAC GCG AGC CGAACGAACAAA | 7 |
| hsa-miR-141-3p | n n n n C CAT CTT TAC CAGACAGTGTTA | 8 |
| hsa-miR-33-3p | n n n n T GCA ATG CAA CTACAATGCAC | 9 |
| miR885-5p | AGCAGCAUUGUACAGGGCUAUCA | 10 |
| miR579-3p | UUCAUUUGGUAUAAACCGCGAUU | 11 |
| miR107 | UUCAUUUGGUAUAAACCGCGAUU | 12 |
| hsa-miR-21-5p | UAGCUUAUCAGACUGAUGUUGA | 13 |
| hsa-miR-301a-3p | CAGUGCAAUAGUAUUGUCAAAGC | 14 |
| hsa-miR-34a-5p | UGGCAGUGUCUUAGCUGGUUGU | 15 |
| hsa-miR-375 | UUUGUUCGUUCGGCUCGCGUGA | 16 |
| hsa-miR-141-3p | UAACACUGUCUGGUAAAGAUGG | 17 |
| hsa-miR-33-3p | GUGCAUUGUAGUUGCAUUGCA | 18 |

After single-stranded oligonucleotide molecule attachment was recognized, PDMS (polydimethylsiloxane) fluidic system was developed for pumping DNA targets to the nanowire surface to hybridize to the DNA probes. Complementary and non-complementary targets were used, with various concentrations with dilution with bis-tris propane [1,3-bis(tris(hydroxymethyl)methylamino)propane] solution. After 30 min for hybridization, samples were washed with bis-tris buffer for 10 min to remove excess targets. Finally, Keithley 2400 was used to detect the NWFET electrical characteristics (Id vs. Vg curves).

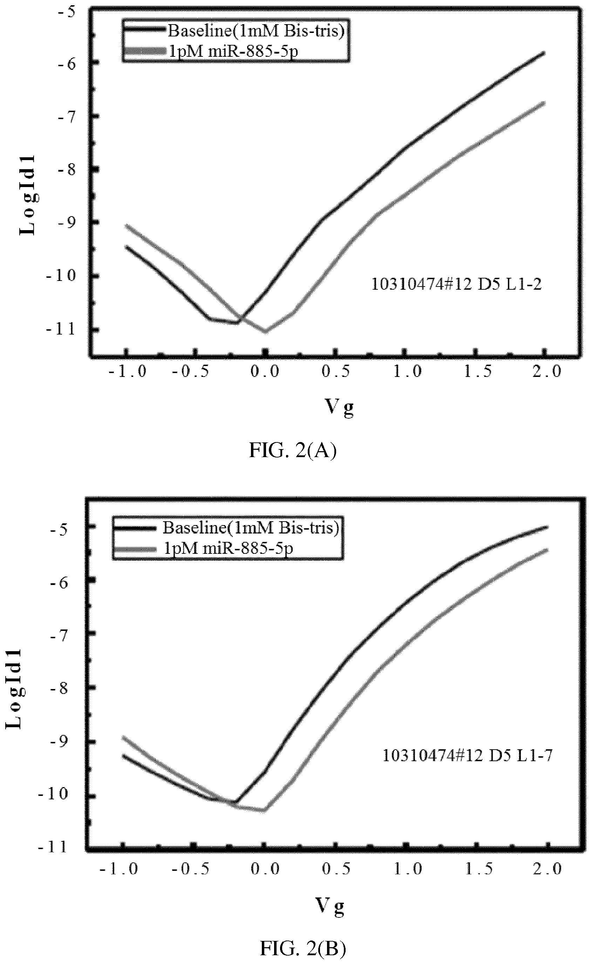

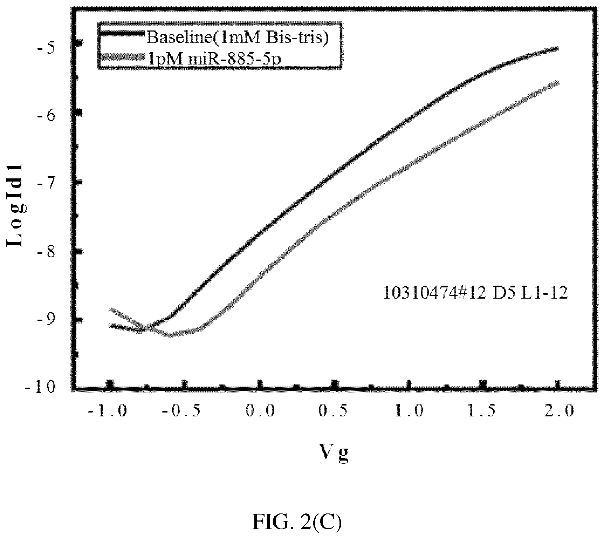

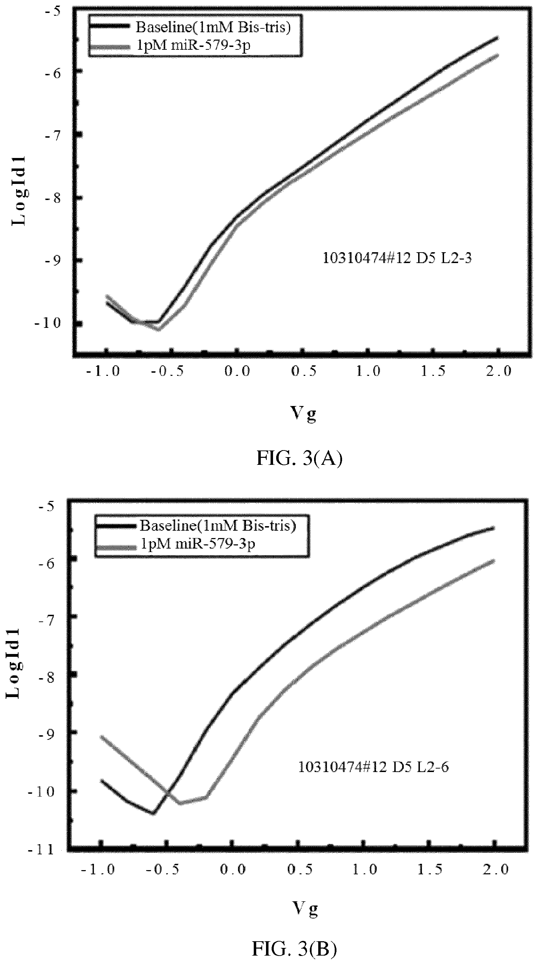

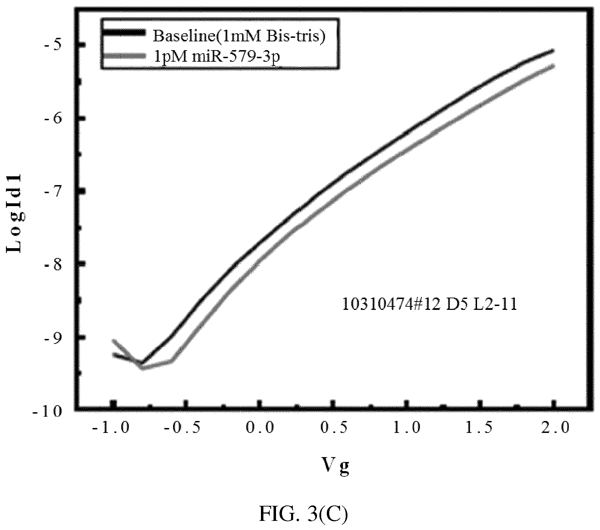

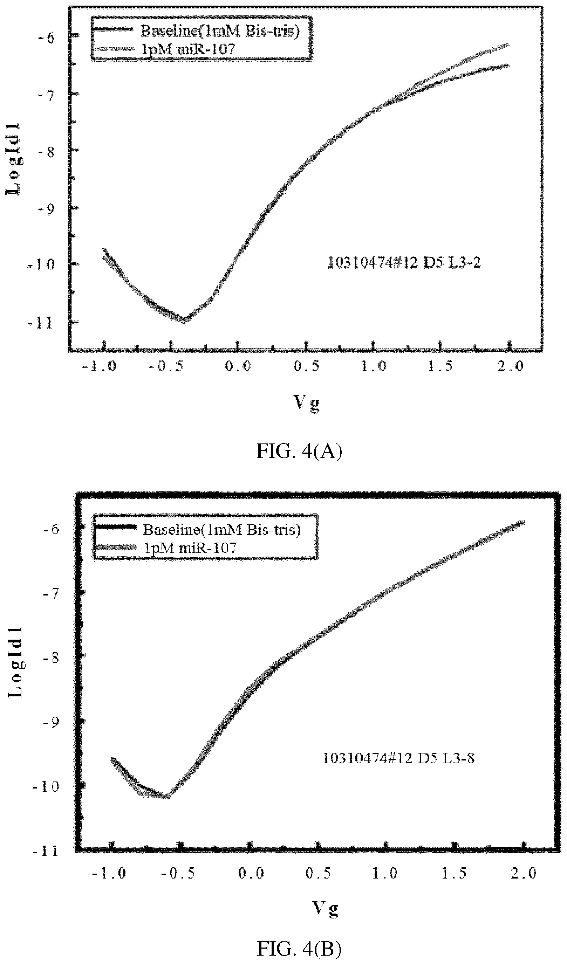

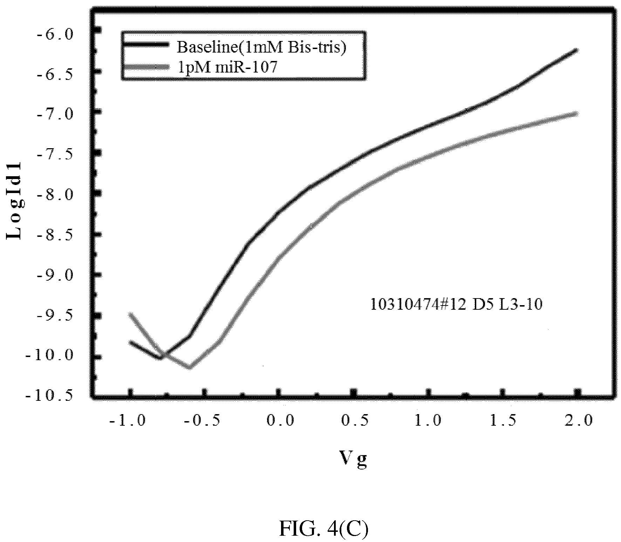

The results of Id-Vg curves as the electrical changes are shown in FIGs. 2 to 5.

Alternatively, after the immobilization of single-stranded oligonucleotides molecule, including neutral DNA, the miRNA targets in cell extracts with 30 ng were directly added on nanowire surface to hybridize with probes, and washed with buffer to remove excess targets and non-specific binding on the probes. Electrical detection devices were used to detect the NWFET electrical characteristics (Id vs. Vg curves). The results of Id-Vg curves, and relative electrical signals were analyzed.

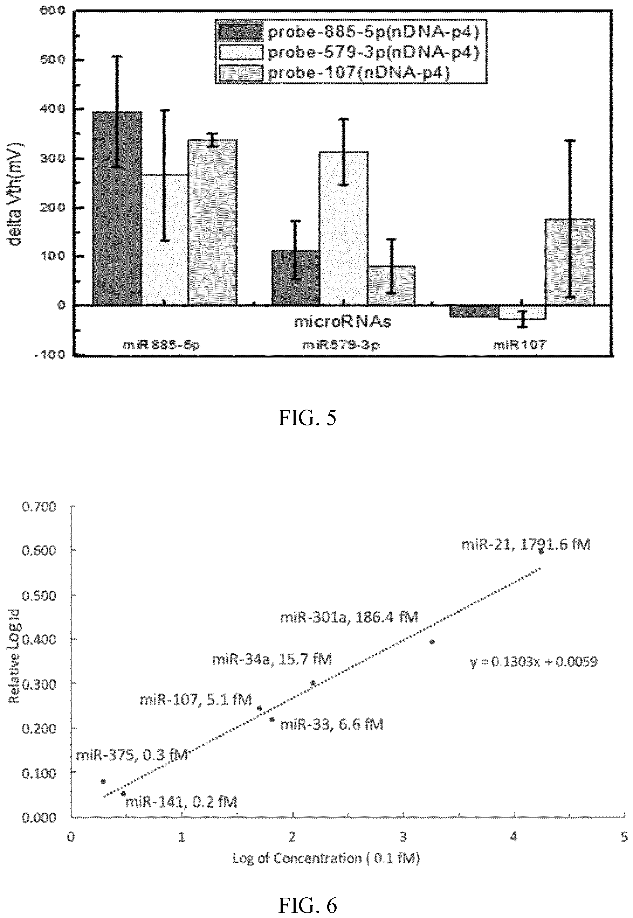

Standard curves were established and compared with what the scientific community generally accepts as a reliable standard, namely, q-PCR, from the same amount of cell extracts, including 30 ng of total RNA extracts from PC3 and CWR cells, respectively. The standard curves are shown in FIGs. 6 to 7.

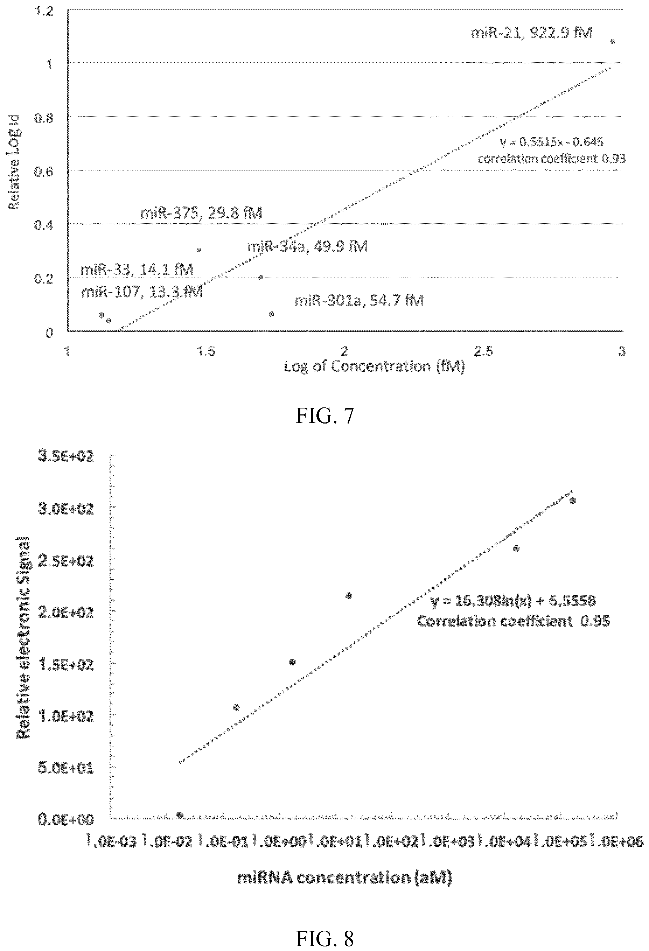

When there is a fair abundance of molecules, standard curves generated by the NWFET device agreed excellently with the q-PCR results. Major deviations appeared when the molecular species became rare. Q-PCR results for extremely rare molecular species are generally regarded as dubious. Whereas, linearity of NWFET signals should at least span four orders of magnitudes, if not more. In FIGs. 8 to 9, the dynamic range of NWFET was spanned for seven orders of magnitudes from 0.0179 fM to 179000 fM by directly adding RNA samples, whereas the dynamic range of q-PCR is limited. In addition, extra procedures are required, including reverse transcription and optimization of q-PCR for trace amount of miRNA. Hence, our current method not only can circumvent amplification and labeling, it offers a far more sensitive detection method and should be particular suited for monitoring subtle changes among rare molecular species, be that miRNA, mRNA, sRNA, lncRNA, etc..

### Consolidating ratios between quantitative signals

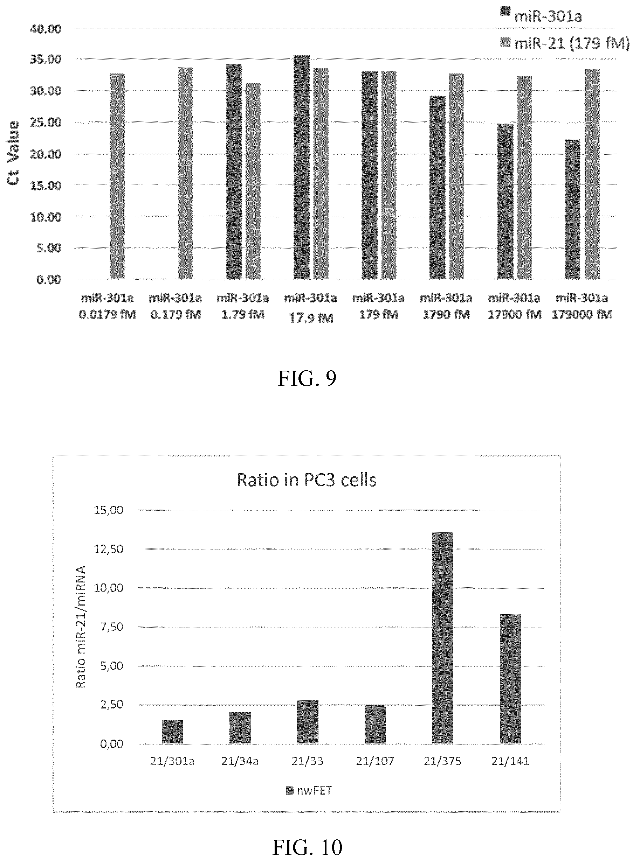

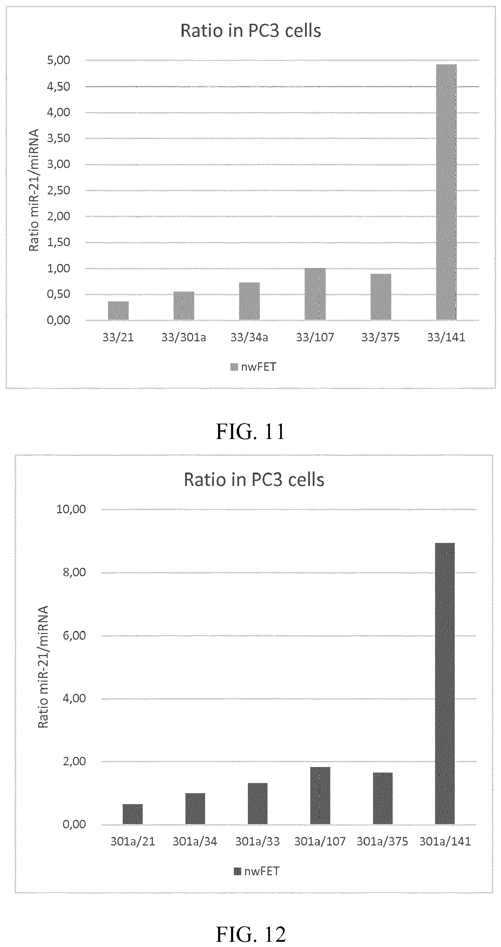

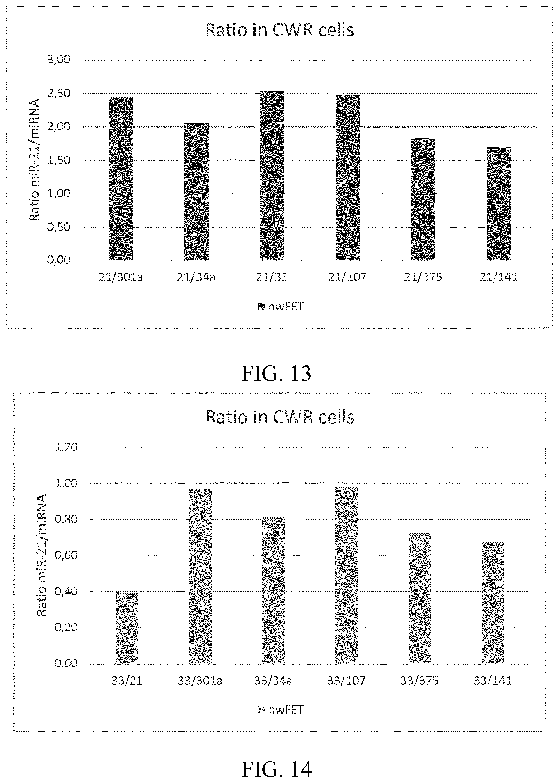

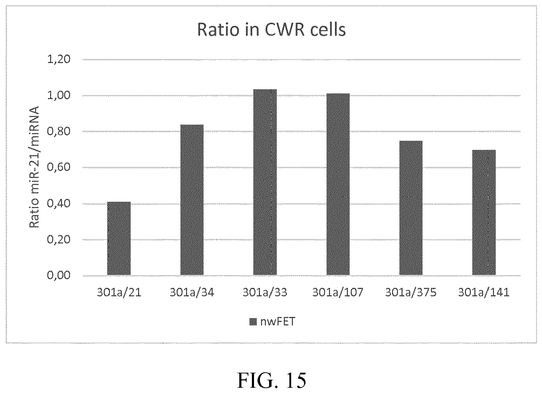

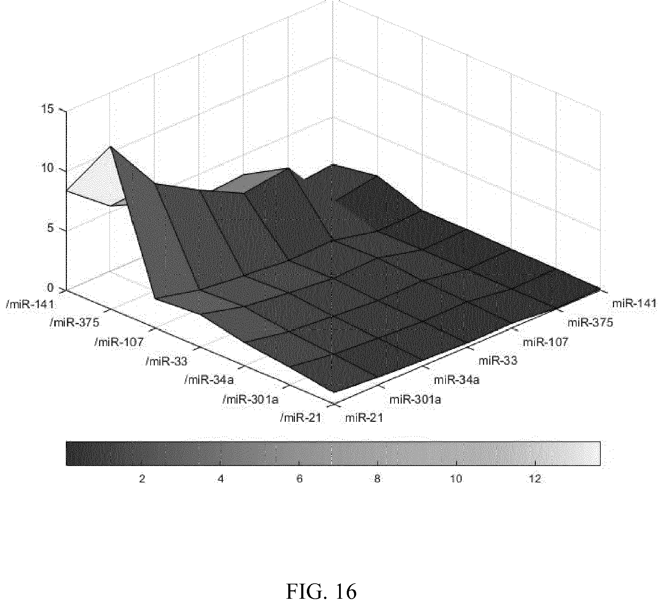

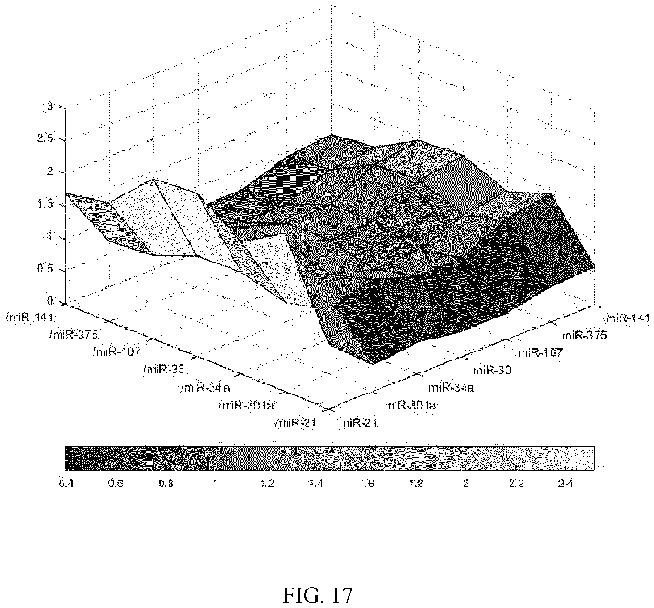

The ratios of the electrical changes are shown in Tables 2 to 7. In addition, the concentration of each miRNA within a cell can be quantified by calibrating against a standard curve. It is equally easy and practical to calculate ratios of concentrations between any two miRNAs, hence, creating a composite miRNA expression landscape for any cells. Data from FIGs. 10 to 15 demonstrates at least 18 set ratio data from 7 miRNA targets for two tumor cell lines: PC3 and CWR cells (both from human prostate cancer) with sub-fM quantitation limit and four order dynamic range. miRNA with 0.01 attomoles was detected within the dynamic range. Comparing the miRNA ratios from the two cancer cell lines showed that the 3D plot of miRNA landscape is not the same even for the same disease (FIGs. 16 and 17). It can only be postulated that the landscapes will also undergo further changes when challenged with different therapeutic agents.

**Table 2:**

| | miR885-5p | miR579-3p | mir107 |
|---|---|---|---|
| Probe-885-5p | 395.44 | 114.26 | -20.99 |
| Probe-579-3p | 266.67 | 314.02 | -27.03 |
| Probe-107 | 338.59 | 81.854 | 177.89 |

**Table 3:**

| | miR885-5p | miR579-3p | mir 107 |
|---|---|---|---|
| Probe(885-5p)/(579-3p) | 1.483 | 0.364 | 0.776 |
| Probe(885-5p)/( 107) | 1.168 | 1.396 | -0.118 |
| Probe(579-3p)/107 | 0.669 | 3.836 | -0.118 |

**Table 4:**

| | Probe(885-5p) | Probe-579-3p | Probe-107 |
|---|---|---|---|
| miR(885-5p)/(579-3p) | 3.461 | 0.849 | 4.136 |
| miR(885-5p)/(107) | -18.839 | -9.866 | 1.903 |
| miR(579-3p)/( 107) | -5.444 | -11.617 | 0.46 |

**Table 5**

| Ratio 21/other miRNA in PC3 cells | | | Ratio 21/other miRNA in CWR cells | | |
|---|---|---|---|---|---|
| miRNA | q-PCR | nwFET | miRNA | q-PCR | nwFET |
| 21/301a | 1.30 | 1.53 | 21/301a | 1.71 | 2.44 |
| 21/34a | 1.94 | 2.01 | 21/34a | 1.75 | 2.05 |
| 21/33 | 2.34 | 2.78 | 21/33 | 2.58 | 2.53 |
| 21/107 | 2.49 | 2.49 | 21/107 | 2.64 | 2.47 |
| 21/375 | 8.91 | 13.61 | 21/375 | 2.01 | 1.82 |
| 21/141 | 14.12 | 8.29 | 21/141 | 1.05 | 1.70 |

**Table 6**

| Ratio 33/other miRNA in PC3 cells | | | Ratio 33/other miRNA in CWR cells | | |
|---|---|---|---|---|---|
| miRNA | q-PCR | nwFET | miRNA | q-PCR | nwFET |
| 33/21 | 0.43 | 0.36 | 33/21 | 0.39 | 0.40 |
| 33/301a | 0.56 | 0.55 | 33/301a | 0.66 | 0.97 |
| 33/34a | 0.83 | 0.73 | 33/34a | 0.68 | 0.81 |
| 33/107 | 1.00 | 1.00 | 33/107 | 1.02 | 0.98 |
| 33/375 | 1.07 | 0.90 | 33/375 | 0.78 | 0.72 |
| 33/141 | 3.81 | 4.92 | 33/141 | 0.41 | 0.67 |

**Table 7**

| Ratio 301a/other miRNA in PC3 cells | | | Ratio 301a/other miRNA in CWR cells | | |
|---|---|---|---|---|---|
| miRNA | q-PCR | nwFET | miRNA | q-PCR | nwFET |
| 301a/21 | 0.77 | 0.66 | 301a/21 | 0.59 | 0.41 |
| 301a/34 | 1.00 | 1.00 | 301a/34 | 1.02 | 0.84 |
| 301a/33 | 1.49 | 1.32 | 301a/33 | 1.51 | 1.03 |
| 301a/107 | 1.80 | 1.82 | 301a/107 | 1.55 | 1.01 |
| 301a/375 | 1.92 | 1.63 | 301a/375 | 1.18 | 0.75 |
| 301a/141 | 6.85 | 8.93 | 301a/141 | 0.62 | 0.70 |

While the present invention has been described in conjunction with the specific embodiments set forth above, many alternatives thereto and modifications and variations thereof will be apparent to those of ordinary skill in the art. All such alternatives, modifications and variations are regarded as falling within the scope of the present invention.

## Claims

1. A method for establishing a quantitative landscape of a group of target nucleic acid molecules, comprising:
conducting a plurality of hybridization reactions for quantifying each target nucleic acid molecule of the group of target nucleic acid molecules to generate a plurality of quantitative signals; wherein the plurality of hybridization reactions have quantitation limits lower than about 1 fM;
generating plural of ratios between any two quantitative signals; and
consolidating the ratios for constructing the quantitative landscape of the group of target nucleic acid molecules.

2. The method according to claim 1, wherein the number of the target nucleic acid molecules in the group of target nucleic acid molecules is *n*, and the number of ratios to be correlated is [*n* x (*n*-1) / 2]; wherein n is an integer.

3. The method according to claim 1 or 2, which is absent from labeling.

4. The method according to one of claims 1 to 3, which is absent from enzyme amplifying.

5. The method according to at least one of claims 1 to 4, wherein the target nucleic acid molecule is selected from the group consisting of DNA, cfDNA, methylated DNA, mRNA, miRNA, LncRNA, and ribosomal RNA.

6. The method according to at least one of claims 1 to 5, wherein the group of target nucleic acid molecules contains at least three target nucleic acid molecules.

7. The method according to at least one of claims 1 to 6, wherein the plurality of hybridization reactions are conducted simultaneously.

8. The method according to at least one of claims 1 to 7, wherein the target nucleic acid molecules are integrated in one chip or microarray.

9. The method according to at least one of claims 1 to 8, wherein the plurality of quantitative signals are selected from the group consisting of an electrical change, a weight change, absorbance wavelength change, absorbance intensity change, fluorescence and fluorescence intensity change, and a reflective index change.

10. The method according to claim 9, wherein the electrical change is selected from the group consisting of an electric charge change, an electric current change, an electric resistance change, a threshold voltage shift change, an electric conductivity change, an electric field change, an electric capacitance change, an electric current change, an electron change, and an electron hole change.

11. The method according to at least one of claims 1 to 10, wherein the plurality of hybridization reactions are conducted with a recognizing single-stranded oligonucleotide molecule attached to a solid surface or the recognizing single-stranded oligonucleotide molecule spaced apart from the solid surface by a distance.

12. The method according to claim 11, wherein the solid surface is a semiconductor-based electrical sensing chip of a field-effect transistor (FET) or a metal surface of a surface plasmon resonance (SPR).

13. The method according to claim 11 or 12, wherein the material of the solid surface is polycrystalline silicon or single crystalline silicon.

14. The method according to at least one of claims 11 to 13, wherein the solid surface is coupled with an electrical change detecting element for detecting the electrical change.

15. The method according to claim 14, wherein the electrical change detecting element is a field-effect transistor or a surface plasmon resonance.

16. The method according to at least one of claims 11 to 15, wherein the recognizing single-stranded oligonucleotide molecule is a partially neutral single-stranded oligonucleotide comprising at least one electrically neutral nucleotide and at least one negatively charged nucleotide.

17. The method according to claim 16, wherein the electrically neutral nucleotide comprises a phosphate group substituted by a C1-C6 alkyl group.

18. The method according to claim 16 or 17, wherein the negatively charged nucleotide comprises an unsubstituted phosphate group.

19. The method according to at least one of claims 16 to 18, wherein the recognizing single-stranded oligonucleotide molecule is attached to a solid surface, and the partially neutral single-stranded oligonucleotide comprises a first portion attached to the solid surface; the length of the first portion is about 50% of the total length of the partially neutral single-stranded oligonucleotide; and the first portion comprises at least one electrically neutral nucleotide and at least one negatively charged nucleotide.

20. The method according to at least one of claims 1 to 19, wherein of the difference between the smallest and largest quantitative signals is at least two orders of magnitude.

21. A quantitative landscape of a group of target nucleic acid molecules, which is established with the method according to one of claims 1 to 20.


SEQUENCE LISTING

<110>  HELIOS BIOELECTRONICS INC.

<120>  METHOD FOR QUANTITATIVELY PROFILING NUCLEIC ACIDS

<130>  NONE

<160>  18

<170>  PatentIn version 3.5

<210>  1
<211>  22
<212>  DNA
<213>  Artificial Sequence

<220>
<223>  Probe


<220>
<221>  misc_feature
<222>  (1)..(1)
<223>  a is neutral

<220>
<221>  misc_feature
<222>  (4)..(4)
<223>  g is neutral

<220>
<221>  misc_feature
<222>  (7)..(7)
<223>  a is neutral

<220>
<221>  misc_feature
<222>  (10)..(10)
<223>  g is neutral

<400>  1
agaggcaggg tagtgtaatg ga                                                22


<210>  2
<211>  22
<212>  DNA
<213>  Artificial Sequence

<220>
<223>  Probe


<220>
<221>  misc_feature
<222>  (1)..(1)
<223>  a is neutral

<220>
<221>  misc_feature
<222>  (4)..(4)
<223>  c is neutral

<220>
<221>  misc_feature
<222>  (7)..(7)
<223>  g is neutral

<220>
<221>  misc_feature
<222>  (10)..(10)
<223>  t is neutral

<400>  2
aatcgcggtt tataccaaat ga                                                22


<210>  3
<211>  23
<212>  DNA
<213>  Artificial Sequence

<220>
<223>  Probe



<220>
<221>  misc_feature
<222>  (1)..(1)
<223>  t is neutral

<220>
<221>  misc_feature
<222>  (4)..(4)
<223>  t is neutral

<220>
<221>  misc_feature
<222>  (7)..(7)
<223>  c is neutral

<220>
<221>  misc_feature
<222>  (10)..(10)
<223>  t is neutral

<400>  3
tgatagccct gtacaatgct gct                                               23


<210>  4
<211>  22
<212>  DNA
<213>  Artificial Sequence

<220>
<223>  Probe



<220>
<221>  misc_feature
<222>  (1)..(1)
<223>  t is neutral

<220>

<221>  misc_feature
<222>  (4)..(4)
<223>  a is neutral

<220>
<221>  misc_feature
<222>  (7)..(7)
<223>  t is neutral

<220>
<221>  misc_feature
<222>  (10)..(10)
<223>  g is neutral

<400>  4
tcaacatcag tctgataagc ta                                                22


<210>  5
<211>  23
<212>  DNA
<213>  Artificial Sequence

<220>
<223>  Probe


<220>
<221>  misc_feature
<222>  (1)..(1)
<223>  g is neutral

<220>
<221>  misc_feature
<222>  (4)..(4)
<223>  t is neutral

<220>
<221>  misc_feature
<222>  (7)..(7)
<223>  a is neutral

<220>
<221>  misc_feature
<222>  (10)..(10)
<223>  a is neutral

<400>  5
gctttgacaa tactattgca ctg                                               23


<210>  6
<211>  22
<212>  DNA
<213>  Artificial Sequence

<220>
<223>  Probe


<220>
<221>  misc_feature
<222>  (1)..(1)

<223>  a is neutral

<220>
<221>  misc_feature
<222>  (4)..(4)
<223>  a is neutral

<220>
<221>  misc_feature
<222>  (7)..(7)
<223>  a is neutral

<400>  6
acaaccagct aagacactgc ca                                                22


<210>  7
<211>  22
<212>  DNA
<213>  Artificial Sequence

<220>
<223>  Probe


<220>
<221>  misc_feature
<222>  (1)..(1)
<223>  t is neutral

<220>
<221>  misc_feature
<222>  (4)..(4)
<223>  c is neutral

<220>
<221>  misc_feature
<222>  (7)..(7)
<223>  g is neutral

<220>
<221>  misc_feature
<222>  (10)..(10)
<223>  c is neutral

<400>  7
tcacgcgagc cgaacgaaca aa                                                22


<210>  8
<211>  22
<212>  DNA
<213>  Artificial Sequence

<220>
<223>  Probe


<220>
<221>  misc_feature
<222>  (1)..(1)
<223>  c is neutral

<220>
<221>  misc_feature
<222>  (4)..(4)
<223>  t is neutral

<220>
<221>  misc_feature
<222>  (7)..(7)
<223>  t is neutral

<220>
<221>  misc_feature
<222>  (10)..(10)
<223>  c is neutral

<400>  8
ccatctttac cagacagtgt ta                                                22

<210>  9
<211>  21
<212>  DNA
<213>  Artificial Sequence

<220>
<223>  Probe

<220>
<221>  misc_feature
<222>  (1)..(1)
<223>  t is neutral

<220>
<221>  misc_feature
<222>  (4)..(4)
<223>  a is neutral

<220>
<221>  misc_feature
<222>  (7)..(7)
<223>  g is neutral

<220>
<221>  misc_feature
<222>  (10)..(10)
<223>  a is neutral

<400>  9
tgcaatgcaa ctacaatgca c                                                 21

<210>  10
<211>  23
<212>  RNA
<213>  Artificial Sequence

<220>
<223>  Target

<400>  10
agcagcauug uacagggcua uca                                               23

<210>  11
<211>  23
<212>  RNA
<213>  Artificial Sequence

<220>
<223>  Target

<400>  11
uucauuuggu auaaaccgcg auu                                              23


<210>  12
<211>  23
<212>  RNA
<213>  Artificial Sequence

<220>
<223>  Target

<400>  12
uucauuuggu auaaaccgcg auu                                              23


<210>  13
<211>  22
<212>  RNA
<213>  Artificial Sequence

<220>
<223>  Target

<400>  13
uagcuuauca gacugauguu ga                                               22


<210>  14
<211>  23
<212>  RNA
<213>  Artificial Sequence

<220>
<223>  Target

<400>  14
cagugcaaua guauugucaa agc                                              23


<210>  15
<211>  22
<212>  RNA
<213>  Artificial Sequence

<220>
<223>  Target

<400>  15
uggcaguguc uuagcugguu gu                                               22


<210>  16
<211>  22

<212>  RNA
<213>  Artificial Sequence

<220>
<223>  Target

<400>  16
uuuguucguu cggcucgcgu ga                                                22


<210>  17
<211>  22
<212>  RNA
<213>  Artificial Sequence

<220>
<223>  Target

<400>  17
uaacacuguc ugguaaagau gg                                                22


<210>  18
<211>  21
<212>  RNA
<213>  Artificial Sequence

<220>
<223>  Target

<400>  18
gugcauugua guugcauugc a                                                 21